# EUROPEAN PATENT SPECIFICATION

(11) **EP 3 496 640 B1**
(45) Date of publication and mention of the grant of the patent: **06.05.2020**
(21) Application number: 16784472.9
(22) Date of filing: 17.10.2016
(51) Int. Cl.: A61B 18/14

(54) **BLOOD-FLOW INTERRUPTING MEANS FOR INSULATING AN IMPLANT DEVICE FOR ABLATION**
VORRICHTUNG ZUR UNTERBRECHUNG DES BLUTFLUSSES FÜR DIE ISOLIERUNG EINES IMPLANTATS ZUR ABLATION
DISPOSITIF D'INTERRUPTION DE FLUX SANGUIN POUR ISOLATION D'UN DISPOSITIF D'IMPLANT POUR ABLATION

(30) Priority: 12.08.2016 EP 16184127; 12.08.2016 EP 16184126
(43) Date of publication of application: 19.06.2019
(73) Proprietor: Medical Development Technologies S.A., 5823 Fentange (LU)
(72) Inventor: VAN LANGENHOVE, Glenn, 9820 Merelbeke (BE)
(74) Representative: Brantsandpatents bvba
(86) International application number: PCT/EP2016/074873
(87) International publication number: WO 2018/028805

(56) References cited:
- WO-A1-01/87174
- WO-A1-99/00064
- WO-A1-2014/197625
- US-A- 6 029 671
- US-A1- 2015 012 069
- Toshiya Kurotobi ET AL: "A case of successful termination of an atrial tachycardia ablated from the pulmonary artery during rapid ventricular pacing", HeartRhythm Case Reports, vol. 1, no. 3, 1 May 2015 (2015-05-01), pages 133-136, XP055651649, ISSN: 2214-0271, DOI: 10.1016/j.hrcr.2015.01.004

## Description

### Technical field

The present invention, which is defined in the appended claims, pertains to the technical field of medical devices and systems.

### Background

Implant devices are well-known medical devices used for the prevention of stenosis and/or the treatment of tissue, for example by ablation. More than one technique exists to make the implant device expand at the treatment zone. In a first alternative, the implant device can be self-expanding. This can be achieved by a combination of constructional and material features. Typically a bio-compatible shape memory alloy such as nitinol is used for the implant device and the implant device comprises zig-zag-like or diamond-shaped structures allowing radial expansion of the implant device. A second alternative is to use an inflatable balloon to make the implant device expand at the treatment zone. Hereby one can insert an uninflated balloon into the compacted implant device and attach this combination to the distal end of a delivery catheter. Then, one can insert the delivery catheter with the combination of the balloon in the implant device into the patient's body and position the implant at the treatment zone. Finally, the balloon can be inflated by controlled providing of pressurized gas to the balloon via a catheter lumen. The inflation of the balloon causes the implant device to expand, allowing it to contact surrounding tissue. Once the implant device is expanded to its desired size, the gas in the balloon can be evacuated via the catheter's lumen, allowing the balloon to shrink and to be removed from the body with the delivery catheter, hereby leaving the implant device in place.

An example of a system and implant device for local treatment is disclosed in document WO 2012/131107. Herein a system is disclosed comprising an implant device and external energy-providing means. The system is particularly developed for ablation of a vessel's wall from the inside, more specifically the system comprises implant devices which can be placed in one or more vessels, preferably in the pulmonary veins for the ablation of the wall of one or more pulmonary veins (PV) from the inside, preferably transmural ablation and preferably at the level of the antrum. Hereby, one or more implant devices can be implanted in the vessels and can subsequently be heated by external energy-providing means. The main objective of the ablation is to cause pulmonary vein isolation (PVI). PVI can solve the problem of atrial fibrillation (AF) by electrically isolating the one or more PVs from the heart tissue such that signals do not travel along myocardial sleeves along the PVs. The document thereto discloses a self-expanding implant device, adapted to be implanted and deployed within said vessel; whereby said implant comprises an ablation region along at least a portion of its length, said ablation region being adapted for surface contact with said vessel and said ablation region subtending at least a substantially complete circumferential band and being effective to ablate tissue within said vessel upon application of energy to the implant to create a signal-blocking path.

By developing an implant device which can be used for PVI according to a system and methods disclosed in WO 2012/131107, the present inventors have found that delivering sufficient energy to the implant device to create the intended lesion for obtaining a signal-blocking path, is cumbersome. In an embodiment of WO 2012/131107, the external energy-providing means comprises an external emitter coil and the implant device comprises a pick-up coil and an ablation region. First, the present inventors have noticed that the power of time-varying magnetic fields emitted by an external emitter coil should be limited in order to prevent skin burns on the skin's surface due to Eddy currents. Second, the alignment of the external coil and the pick-up coil is difficult, the imperfect alignment further increasing the loss of emitted time-varying magnetic fields in body tissue. Third, time-varying magnetic fields which are coupled from the emitter coil to the pick-up coil are converted into heat in the ablation region of the implant device by Joule heating, but a very large portion of the heat at the ablation region and/or the treatment zone is drained by the blood flow due to convective heat transfer. Hereby, the blood flow is largely dependent on the physiology of the patient, i.e. each patient has a unique blood flow pattern at the region of the treatment zone, which can moreover change over time, even during any procedure as e.g. disclosed in WO 2012/131107.

Document US 8,465,481 discusses a cryotherapy catheter including an elongate member and an inflatable balloon portion at a distal end of the elongate member. The inflatable balloon portion has an external surface and an internal chamber, and the external surface includes a cooling region and a thermally insulated region. The internal chamber is configured to receive during a cryotherapy procedure a cryogenic agent for extracting heat from body tissue that is in contact with the cooling region. A thermal profiling component is disposed in the interior chamber and configured to thermally insulate the thermally insulated region from the cryogenic agent to minimize heat extraction by the cryogenic agent from body tissue that is in contact with the thermally insulated region. The cryotherapy catheter delivers cryotherapy to ablate tissue by freezing it.

Document WO 1991/000118 discusses a balloon catheter for canalizing occluded blood vessels comprising a catheter tube, a longitudinally extending balloon unit having a longitudinally extending cylindrical section and two end sections. The invention is characterized by heat insulating means provided at the end sections of the balloon unit as well as by the fact that the cylindrical section is manufactured of heat-conducting material. Hot liquid, possibly with the addition of X-ray contrast material, is introduced through a lumen to the internal space enabling the wall surface of a blood vessel to be exposed to thermal action.

Document US 7,371,235 discusses an ablation system comprising a support body, an energy transmitting element supported by the support body and an insulating member covering a portion of the support body and energy transmitting member. In an embodiment, the support body further comprises an inflatable insulation balloon to insulate the energy transmitting element and the tissue at the ablation center from the blood flow to substantially prevent convective transport of heat away from the ablation site.

The system disclosed in document WO 2012/131107 has the disadvantage that heat is drained from the ablation region of the implant device and/or the treatment zone by convective heat transfer due to the blood flow. The system disclosed in document US 7,371,235 has three disadvantages. A first disadvantage is the need to direct the energy transmitting element at the distal end of the support body at least substantially along the circumference of the vessel to ablate a lesion which blocks all signals. A second disadvantage is the inability of the system to prevent stenosis after the ablation. A third disadvantage is the difficulty to form a new lesion at a different moment in time at or substantially close to an older lesion, due to the need to redirect the energy transmitting element substantially close to the older lesion. A device according to the preamble of claim 1 is known from Toshiya Kurotobi ET AL: "A case of successful termination of an atrial tachycardia ablated from the pulmonary artery during rapid ventricular pacing", HeartRhythm Case Reports, vol. 1, no. 3, 1 May 2015.

The present invention aims to resolve at least some of the problems mentioned above.

### Summary of the invention

The present disclosure pertains to a system for ablating tissue to create a signal-blocking path on an inner wall of a vessel, comprising:
- an implant device provided with an ablation region which is adapted for surface contact with the inner wall of the vessel, the ablation region adapted for subtending at least a substantially complete circumferential band for ablating tissue to create a signal-blocking path at a treatment zone in the vessel upon heating of the ablation region;
- energy-providing means configured to provide the implant device with energy for heating up the ablation region,
**characterized in that,** the system comprises:
- blood-flow interrupting means for temporarily interrupting blood from flowing along the ablation region of the implant device and/or along the treatment zone, thereby limiting heat being transferred away from the ablation region and/or treatment zone.

To reduce the cooling effect of the blood, the inventors have found that it is necessary to reduce the thermal contact between the blood flow and the treatment zone and/or ablation region. This can be achieved by blood-flow interrupting means which alter the blood flow during the treatment in such a way that cooling of the ablation region or treatment zone by the blood flow is significantly reduced. The blood-flow interrupting means can hereby alter the blood flow by temporarily stopping the blood-flow during the ablation treatment, preferably the blood flow being stopped, preferably upstream of the treatment zone and optionally also downstream of the treatment zone, and/or by re-routing the blood flow along a path which is thermally isolated from the ablation region and/or the treatment zone during the ablation treatment.

The present system is further advantageous because the implant device is provided with an ablation region which upon placement of the implant device makes contact with the inner wall of the vessel along at least a substantially complete circumferential band. No redirection of an energy emitting element is hence required to obtain a full signal-blocking path, as the ablation region of the implant device subtends upon a single placement of the implant device a substantially complete circumferential band for ablating tissue to create a signal-blocking path. Furthermore, the implant device remains in the vessel, which prevents stenosis, and which allows to create a second lesion at a different moment in time substantially close to a first lesion.

The inventors have also found a number of preferred embodiments which are discussed below.

### Description of figures

**Figures 1** **and** **2** are schematic representations of embodiments of the system of the present invention.
**Figures 3 and 4** are schematic representations of an implant device and the distal end of a balloon catheter according to embodiments of the system of the present invention.
**Figure 5** is a schematic representation of an implant device in a compacted state, a distal end of a balloon catheter inserted in the implant device in the compacted state, and protector sheath covering the implant device.

### Detailed description of the invention

The present disclosure concerns a system for ablating tissue to create a signal-blocking path on an inner wall of a vessel, comprising an implant device provided with an ablation region; energy-providing means configured to provide the implant device with energy for heating up the ablation region; and blood-flow interrupting means for temporarily interrupting blood from flowing along the ablation region of the implant device and/or along the treatment zone, thereby limiting heat being transferred away from the ablation region and/or treatment zone.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings: "A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The present disclosure pertains to a system for ablating tissue to create a signal-blocking path on an inner wall of a vessel, comprising:
- an implant device provided with an ablation region which is adapted for surface contact with the inner wall of the vessel, the ablation region adapted for subtending at least a substantially complete circumferential band for ablating tissue to create a signal-blocking path at a treatment zone in the vessel upon heating of the ablation region;
- energy-providing means configured to provide the implant device with energy for heating up the ablation region,
**characterized in that,** the system comprises:
- blood-flow interrupting means for temporarily interrupting blood from flowing along the ablation region of the implant device and/or along the treatment zone, thereby limiting heat being transferred away from the ablation region and/or treatment zone.

The system of the present invention is therefore able to significantly reduce the drain of heat from the ablation region and/or the treatment zone due to forced convection of the blood flow. In addition, the system of the present invention is further advantageous due to the implant device. First, the implant device comprises an ablation region adapted for subtending a substantially complete circumferential band in contact with the vessel, thereby avoiding the need to position a catheter tip with an energy emitting element for ablation consecutively along multiple closely spaced positions along the vessel wall for ablating a circumferential lesion to create a signal-blocking path. Second, as the implant device remains in the vessel after treatment, it prevents stenosis. Third, as the implant device remains in the same position in the vessel after treatment, creating a new lesion substantially close to an older lesion at a different moment in time is substantially easier than with said catheter tip with energy emitting element.

In a preferred embodiment of the system of the present disclosure, the blood-flow interrupting means comprises a pressurizing means and a balloon catheter, the balloon catheter comprising:
- a distal end, a proximal end, and a longitudinal body in between;
- one or more inflatable balloons at or near the distal end of the balloon catheter, each of the balloons comprising:
   - an evacuated balloon state adapted for insertion into the vessel;
   - an expanded balloon state adapted for at least partially closing off the vessel;
   - an exterior balloon surface; and
   - an interior balloon chamber;
- one or more inflation lumens inside the longitudinal body, each of the inflation lumens extending from the distal end to the proximal end of the balloon catheter, whereby each of the inflation lumens is at the distal end of the balloon catheter in communication with the interior balloon chamber of at least one balloon, and whereby the inflation lumens are adapted for connection with the pressurizing means at the proximal end.

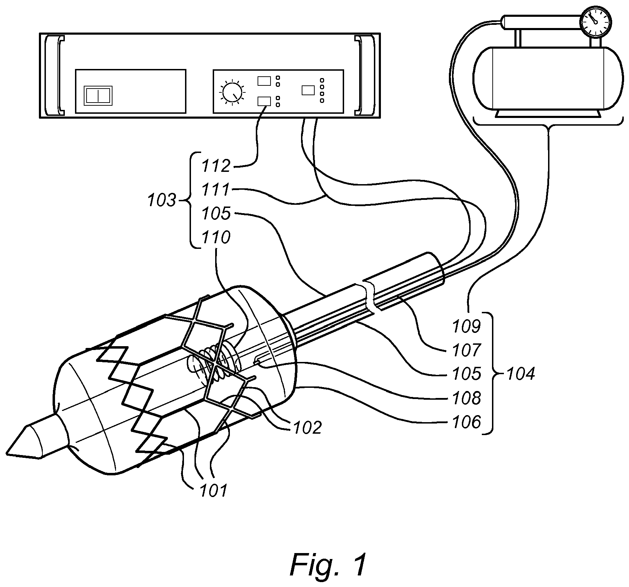

**FIG. 1** is a schematic representation of a preferred embodiment of the system of the present invention comprising an implant device **101,** an energy-providing means **103**, and a blood-flow interrupting means **104**. The implant device **101** comprises an ablation region **102**, adapted for subtending at least a substantially complete circumferential band in contact with the vessel wall for ablating tissue to create a signal-blocking path. For insertion in the vessel and subsequent deployment, the implant device **101** comprises a compacted state and an expanded state, and during deployment it should be expandable from the compacted state to the expanded state. This can be realized by zig-zag-like or diamond-shaped elements, which allow for radial expansion. The implant device **101** comprises two ring structures of diamond-shaped elements, interconnected with a set of struts. One of the two ring structures, namely ring structure **102**, comprises the ablation region of the implant device. The particular embodiment of the implant device in **FIG. 1** should not be interpreted as limiting.

In another embodiment, the implant device can consist of only a single ring structure of diamond-shaped or zig-zag-like elements, the single ring structure comprising the ablation region. The implant device can also comprise anchoring means for attachment at or near the treatment zone in the vessel. Furthermore, the implant device can be self-expandable from the compacted to the expanded state, for example when it comprises a biocompatible shape memory alloy such as nitinol. The implant device can also be non-self-expandable, requiring an additional means for expanding the implant device from the compacted to the expanded state. The ring structures of the implant device can be circular. The ring structures of the implant device can also be oval. The implant device can also comprise multiple ring structures of different size. The implant device can be adapted for positioning the implant device in a pulmonary vein at or near a treatment zone, more specifically in the antrum or ostium of the pulmonary veins. The implant device may be characterized in that the greatest distance between two points that can be measured on any of the circular or oval ring structures will range from 3 to 30 mm, more specifically from 5 mm to 20 mm, even more specifically from 9 mm to 13 mm, if to be implanted at the ostia of the pulmonary vein. The implant device may be characterized in that the greatest distance between two points that can be measured on any of the circular or oval ring structures will range from 5 to 50 mm, more specifically from 8 mm to 40 mm, even more specifically from 10 mm to 30 mm, if to be implanted at the site of the antrum.

The implant device can be fully or partially coated with a thermoactive coating with an activation temperature above a predefined temperature, preferably above 37°C, more preferably above 40°C, such as 41°C, 42°C, 43°C, 44°C, 45°C, to activate the thermoactive coating simultaneous to and/or after heating of the ablation region. The thermoactive coating can comprise substances capable of producing a lesion of limited necrosis and/or neurotoxicity. The implant device can further also comprise small chambers and/or pockets and/or another coating comprising substances which are released upon or after activation of the thermoactive coating.

The energy-providing means of the system should be able to provide the implant device with energy for heating the ablation region. In the particular embodiment in **FIG. 1** the energy-providing means **103** comprises an emitter coil **110** at the distal end of a coil catheter **105**. The coil catheter **105** further comprises electrical wiring **111** in its longitudinal body, extending from the distal end of the coil catheter to the proximal end, connected with the emitter coil **110** at the distal end, and connectable at the proximal end with an electric power source **112**. The electric power source **112** is adapted to provide via the electrical wiring **111** the emitter coil **110** with an alternating current, which is converted in the emitter coil **110** to a time-varying magnetic field. This time-varying magnetic field is picked-up by a pick-up coil **102** of the implant device **101**, which in the particular embodiment of **FIG. 1** comprises the ablation region **102**. The time-varying magnetic field induces a current in the pick-up coil **102** of the implant device **101**, which is converted in the ablation region **102** into heat. The ablation region **102** can comprise a material with higher resistivity in which the current is converted into heat by Joule heating. During treatment, the ablation region is heated to a temperature which is high enough to cause lesions to the tissue nearby the ablation region, i.e. in the treatment zone, and thereby create a, preferably transmural, circumferential lesion which results in a signal-blocking path on the vessel. The particular embodiment of the energy-providing means **103** in **FIG. 1** should not be interpreted as limiting.

In another embodiment, the energy-providing means can comprise an emitter coil which is adapted for emitting time-varying magnetic fields from a position outside a patient's body to the pick-up coil of an implant device near the treatment zone inside the vessel in the patient's body. The energy-providing means should also not be interpreted as limited to the transfer of time-varying magnetic fields from an emitter coil to a pick-up coil. In another embodiment, the implant device can be deployed with a placement catheter. The placement catheter comprises a distal end, a proximal end, and a longitudinal body in between. The longitudinal body of the placement catheter can comprise wiring which is connected at the distal end to the implant device in the compacted state, and which is connectable at the proximal end to an electric power source. After insertion of the placement catheter into a patient's body and positioning of the distal end of the catheter in the vessel near the treatment zone, the implant device can be deployed from the compacted to the expanded state. When the implant device is self-expandable, this can be performed by shielding the implant device during insertion with a movable protector sheath, and retracting the movable protector sheath when the implant device is at the desired position at or near the treatment zone in the vessel. When the implant device is not self-expandable, this can be performed with an inflation balloon to which the implant device is attached upon insertion of the placement catheter into the patient's body, and which can be inflated once the implant device is at the desired position near the treatment zone in the vessel. Once the implant device is deployed, the ablation region can be heated by providing a current to the ablation region of the implant device with the electric power source via the electrical wiring inside the placement catheter, thereby heating the ablation region due to Joule heating due to the current. Once the lesion is ablated which creates a signal-blocking path, the wiring can be detached from the implant device, withdrawn inside the catheter, and subsequently the catheter can be removed from the patient's body.

The blood-flow interrupting means should be able to temporarily interrupt or limit blood from flowing along the ablation region of the implant device and/or along the treatment zone, thereby limiting heat being transferred away from the ablation region and/or treatment zone. In the particular embodiment of **FIG. 1**, the blood-flow interrupting means **104** comprises a balloon catheter **105**, comprising an inflatable balloon **106** at its distal end, the inflatable balloon **106** comprising an evacuated and an expanded balloon state, the inflatable balloon further comprising an interior balloon chamber and an exterior balloon surface. The balloon catheter **105** further comprises an inflation lumen **107** extending from the distal end to the proximal end of the balloon catheter **105**. The interior balloon chamber is in communication with the inflation lumen **107** by means of an opening **108** in the catheter shaft. The inflation lumen **107** can be connected at the proximal end of the balloon catheter **105** to a pressurizing means **109**, adapted to inflate the balloon **106** from the evacuated balloon state to the expanded balloon state. In the preferred embodiment in **FIG. 1**, the balloon catheter **105** fully coincides with the coil catheter **105**, preferably the balloon catheter **105** fully coincides with the coil catheter **105** with the emitter coil **110** positioned in the immediate vicinity of the balloon **106**, even more preferably the balloon catheter **105** fully coincides with the coil catheter **105** with the balloon **106** fully surrounding the emitter coil. The particular embodiment of the blood-flow interrupting means in **FIG. 1** should not be interpreted as limiting. In another embodiment, the balloon catheter can partially coincide with the coil catheter. In yet another embodiment, the balloon and coil catheters do not coincide at all. The balloon **106** can in its evacuated balloon state be inserted in a patient's body near the treatment zone in the vessel by means of the balloon catheter **105**. The balloon **106** can then be inflated with a pressurizing means **109** in communication with the balloon **106** via the inflation lumen **107** in order to temporarily interrupt or limit the blood flow completely. When the ablation region **102** of the implant device **101** is heated, the drain of heat by forced convection due to the blood flow is limited, as the blood flow is interrupted or limited. The balloon **106** can be placed upstream of the ablation region **102,** at the ablation region **102,** and/or downstream of the ablation region **102.** When the balloon **106** is placed at the ablation region **102,** the balloon surface is in contact with the ablation region **102.** It can thereby thermally insulate the ablation region **102** and/or the treatment zone from blood. To substantially insulate the ablation region **102** from the blood, a part of the balloon **106** can be placed upstream of the ablation region **102** and a part of the balloon **106** can be placed downstream of the ablation region **102.** When the energy-providing means **103** comprises a coil catheter **105** which coincides with the balloon catheter **105,** as in the embodiment in **FIG. 1**, the inflatable balloon **106** can be placed in the immediate vicinity of the emitter coil **110**, more preferably the balloon **106** surrounds the emitter coil **110**. When the balloon **106** surrounds the emitter coil **110** and the balloon **106** is placed at the ablation region **102**, the emitter coil **110** is placed at, in, or near the pick-up coil **102** of the implant device **101**, ensuring an optimal coupling of electromagnetic fields from the emitter coil **110** to the pick-up coil **102**. The particular embodiment of the blood-flow interrupting means in **FIG. 1** should not be interpreted as limiting.

Alternatively, or in addition to a balloon adapted to interrupt or limit the blood flow, the blood-flow interrupting means may comprise rapid pacing means for interrupting the blood flow.

Rapid pacing or rapid ventricular pacing causes the heart to beat at approximately 200 to 400 beats/min, which functionally means that the heart is in continuous contraction, never fills with blood between beats, and therefore the flow of blood through the heart is stopped. Rapid ventricular pacing produces a heart that is in contractile, rigid arrest. Rapid ventricular pacing is functionally "systolic arrest".

Rapid pacing means can be any type of means for inducing rapid pacing. Typically, the rapid pacing means comprise one or more cardiac catheters with one or more electrodes near a distal end thereof for providing electrical stimuli to the heart, preferably to the left atrium and/or left ventricle. Typically, at least a first cardiac catheter is used to provide electrical stimuli to the left atrium to induce rapid pacing.

In a preferred embodiment of the system of the present invention, the blood-flow interrupting means comprises, either in place of or in addition to the pressurizing means and the balloon catheter, a rapid pacing means comprising a pacemaker and one or more pacing catheters, each of the pacing catheters comprising:
- a distal end, a proximal end, and a longitudinal body in between;
- electrical wiring in the longitudinal body of the pacing catheter extending from the distal end to the proximal end, and adapted at the proximal end for connection to the pacemaker; and
- one or more electrodes at or near the distal end of the pacing catheter, connected at or near the distal end of the pacing catheter to the electrical wiring,
and whereby the pacemaker comprises a pacing output circuit adapted to deliver pacing pulses through at least one of the electrodes of at least one of the pacing catheters, when the pacing output circuit is connected to the electrical wiring of at least one of the pacing catheters at its proximal end.

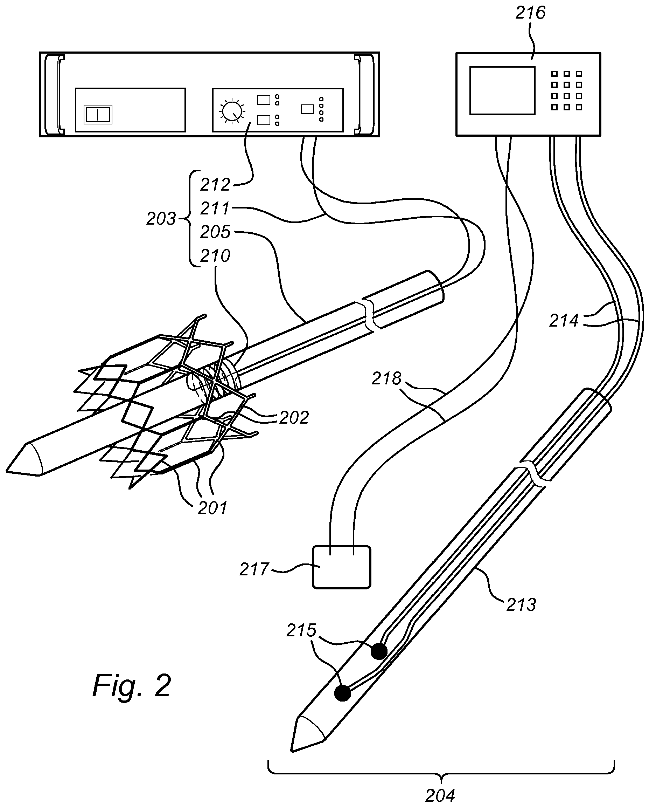

**FIG. 2** is a schematic representation of another preferred embodiment of the system of the present invention comprising an implant device **201**, an energy-providing means **203**, and a blood-flow interrupting means **204**. The particular implant device depicted in **FIG. 2** comprises the same components as the implant device in **FIG. 1**, with the same functions, and can be interpreted in a similar way, including the notion that the implant device is not limited to the particular embodiment of **FIG. 2**. The particular energy-providing means depicted in **FIG. 2** comprises the same components as the energy-providing means in **FIG. 1**, with the same functions, and can be interpreted in a similar way, including the notion that the energy-providing means is not limited to the particular embodiment of **FIG. 2**. The blood-flow interrupting means in **FIG. 2** comprises a pacemaker **216**, a pacing catheter **213**, and a skin patch electrode **217**. The pacing catheter **213** comprises two electrodes **215** at or near its distal end and electrical wiring **214** extending from the distal end to the proximal end of the pacing catheter **213**. The electrical wiring **214** is connected at or near the distal end of the pacing catheter **213** with the electrodes **215** and can be connected to the pacemaker **216** at or near the proximal end of the pacing catheter **213**. The skin patch electrode **217** is connectable via electrical wiring **218** to the pacemaker **216**, and can be used as reference electrode during rapid pacing. The pacemaker **216** comprises a pacing output circuit adapted to deliver pacing pulses through the electrodes **215** of the pacing catheter via the electrical wiring **214**. The particular embodiment of the blood-flow interrupting means in **FIG. 2** should not be interpreted as limiting.

In another embodiment, the rapid-pacing means can only comprise the pacemaker and the pacing catheter, while the skin patch electrode is not present. In another embodiment, the rapid-pacing means can comprise multiple pacing catheters, some of which can partially coincide. In another embodiment, one of the pacing catheters can coincide partially with the coil catheter and/or the balloon catheter, when present. In yet another embodiment, the rapid pacing means do not comprise pacing catheters.

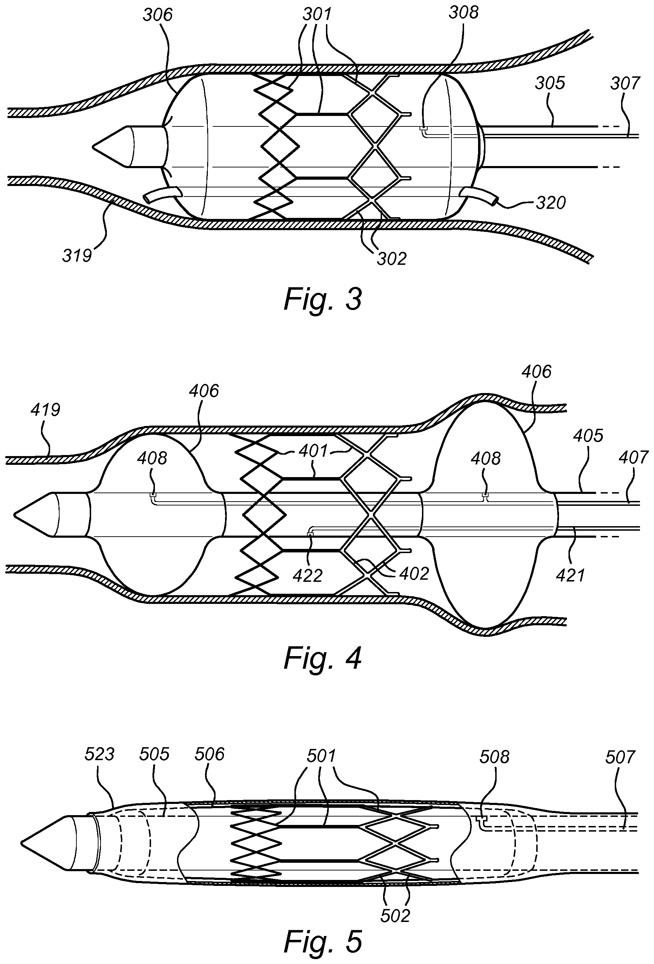

We now turn to particular embodiments of the balloon catheter. **FIG. 3** is a schematic representation of an implant device **301** and the distal end of a balloon catheter **305**, according to a preferred embodiment of the present invention, both inserted at the treatment zone in the vessel **319** in a patient's body. The implant device **301** comprises an ablation region **302**, which makes substantially complete circumferential surface contact with the inner wall of the vessel. The particular implant device depicted in **FIG. 3** comprises the same components as the implant device in **FIG. 1**, with the same functions, and can be interpreted in a similar way, including the notion that the implant device is not limited to the particular embodiment of **FIG. 3**. A balloon catheter **305** can be used to insert a balloon **306** in its evacuated state into the vessel **319**, at a position at, in, or near the implant device **301**. When the distal end of the balloon catheter **305** is positioned as desired, the balloon **306** can be inflated with a pressurizing means via an inflation lumen **307** inside the catheter's body, which is in communication with the balloon **306** via an opening **308** in the catheter shaft. The exterior balloon surface of the balloon **306** is adapted for circumferential surface contact with the implant device **301** and/or the wall of the vessel **319**, thereby substantially preventing or limiting blood from reaching the ablation region **302** of the implant device **301** and/or the treatment zone of the vessel **319**. In **FIG. 3**, the inflated balloon **306** is positioned at the ablation region of the implant device, as well as partly upstream and partly downstream of the implant device. In the particular embodiment in **FIG. 3**, the balloon **306** further comprises a perfusion lumen **320**. A balloon comprising one or multiple perfusion lumens is called a perfusion balloon in the art. With the perfusion lumen **320**, limited blood flow can still be sustained, thereby significantly diminishing the chance of blood clots, while the ablation region **302** of the implant device **301** and/or the treatment zone are thermally insulated from the blood flow due to the balloon **106**. The particular embodiment of the distal end of the balloon catheter **305** should not be interpreted as limiting.

In another embodiment, the balloon can comprise no perfusion lumen, thereby fully closing off the vessel when the balloon is in the expanded state, and realizing a substantially complete stop of blood flow in the vessel. The balloon can thereby also comprise an anticoagulant coating to prevent the formation of blood clots. In yet another embodiment, the balloon can comprise multiple perfusion lumens. A balloon with two or more perfusion lumens is also called a perfusion balloon. Another way to sustain a limited blood flow is by means of a perfusion lumen in the balloon catheter, in communication with the region upstream of the inflated balloon by means of at least one opening in the catheter shaft, and in communication with the region downstream of the inflated balloon by means of at least one opening in the catheter shaft. In another embodiment, now referring to **FIG. 1**, the system of the present invention can also comprise a coil catheter, which can fully coincide with the balloon catheter, preferably with the coil surrounded by the balloon, and the coil located in a position in, at, or near the implant device, preferably in, at, or near the pick-up coil of the implant device.

In yet another embodiment, the balloon catheter comprises two or more balloons at its distal end. We now turn to **FIG. 4**. The figure is a schematic representation of an implant device **401** and the distal end of a balloon catheter **405**, according to a preferred embodiment of the present invention, both inserted at the treatment zone in the vessel **419** in a patient's body. The implant device **401** comprises an ablation region **402**, which makes substantially complete circumferential surface contact with the inner wall of the vessel. The particular implant device depicted in **FIG. 4** comprises the same components as the implant device in **FIG. 1**, with the same functions, and can be interpreted in a similar way, including the notion that the implant device is not limited to the particular embodiment of **FIG. 4**. The balloon catheter **405** comprises two balloons **406** in communication with an inflation lumen **407** by means of openings **408** in the catheter shaft, the inflation lumen further adapted for connection with a pressurizing means to inflate the balloons **406**. The inflated balloons **406** close off the vessel at least substantially complete at two different locations. A first balloon is located upstream of the implant device **401**, and a second balloon is located downstream of the implant device **401**. This prevents blood flow along the ablation region **402** of the implant device **401** and/or along the treatment zone. The blood in the section of the vessel **419** closed off by the two balloons **420** can be fully or partially replaced with another substance, or another substance can be added to said section, by means of a fluid lumen **421** in communication with said section by means of an opening **422** in the catheter shaft. The substance can comprise an anticoagulant to prevent the formation of blood clots in said section. The particular embodiment of the distal end of the balloon catheter **405** should not be interpreted as limiting.

In another embodiment, the balloon catheter can comprise more than two balloons. In yet another embodiment, different balloons can be in contact with the same inflation lumen or with different inflation lumens. One or more of the balloons can also comprise an anticoagulant coating, preferably heparin sulphate. In another embodiment, the system of the present invention can also comprise a coil catheter, which can fully coincide with the balloon catheter, preferably with the coil located in a position in, at, or near the implant device, preferably in, at, or near the pick-up coil of the implant device.

We now turn to **FIG. 5**. The figure is a schematic representation of an implant device **501** in a compacted state and a distal end of a balloon catheter **505** inserted in the implant device **501**. The balloon catheter **505** comprises a protector sheath **523** covering the implant device **501**. The distal end of the balloon catheter in **FIG. 5** can be positioned at or near the treatment zone in the vessel of a patient's body by means of the balloon catheter. The protector sheath **523** can be withdrawn, thereby uncovering the implant device **501**. Uncovering a self-expandable implant device **501** allows for its radial expansion from the compacted state to the expanded state, thereby positioning the implant device **501** in the vessel whereby the ablation region **502** of the implant device **501** subtends substantially complete circumferential surface contact with the inner wall of the vessel. A non-self-expandable implant device **501** can be radially expanded from its compacted state to its expanded state by means of an inflatable balloon **506**. The balloon **506** is in communication with an inflation lumen **507** by means of an opening **508** in the catheter shaft. The inflation lumen **507** can be connectable to a pressurizing means to inflate the balloon **506**. Upon inflation of the balloon **506**, the implant device **506** is expanded and thereby deployed to the vessel's inner wall. The same balloon **506** can be utilized to close off the vessel, and/or to thermally insulate the ablation region **502** of the implant device **501** and/or the treatment zone from the blood flow in the vessel.

The invention is further described by the following non-limiting examples which further illustrate the invention, and are not intended to, nor should they be interpreted to, limit the scope of the invention.

### Examples

### Example 1: Pulmonary vein isolation

The myocardium is a conductive heart tissue for pacemaker activity. Atrial fibrillation is a cardiac condition characterized by rapid and irregular beating, due to rapid firing of myocardial sleeves extending onto the pulmonary veins. A treatment of atrial fibrillation is the creation of a signal-blocking path at the level of the antrum of the pulmonary vein by ablation, to electrically insulate the myocardial sleeves from the remainder of the myocardium. The treatment is called pulmonary vein isolation. This example illustrates how the system of the present invention can be used to create pulmonary vein isolation.

To this end, we consider an embodiment of the system of the present invention, comprising:
- a substantially cylindrical self-expandable implant device **301** comprising:
   - two ring structures comprising diamond-shaped elements, interconnected by means of struts;
   - an implant central axis, coinciding with the rotation axis of the cylindrically shaped implant device **301**, and parallel to the struts;
   - an ablation region **302** which is adapted for surface contact with the inner wall of the pulmonary vein **319** at the level of the antrum, the ablation region **302** adapted for subtending at least a substantially complete circumferential band for ablating tissue to create a signal-blocking path at a treatment zone in the pulmonary vein **319** upon heating of the ablation region **302**;
   - a compacted state for insertion in the pulmonary vein (**FIG. 5**);
   - an expanded state for attachment of the implant device to the inner wall of the pulmonary vein **319** at the level of the antrum;
   - a pick-up coil **102** comprising the ablation region **102** which is adapted to pick-up time varying magnetic fields from an emitter coil **110** inserted in the implant device **101**, whereby the axis of the emitter coil is preferably aligned substantially coaxial with the implant central axis,
- an energy-providing and blood-flow interrupting means **103**, **104** configured to provide the implant device **101** with energy for heating up the ablation region **102** and to prevent or limit forced convection of heat from the ablation region **102** of the implant device **101** and/or the treatment zone in the pulmonary vein due to blood flow, comprising an electric power source **112**, a pressurizing means **109**, and a catheter **105**, the catheter **105** comprising:
   - a distal end, a proximal end, and a longitudinal body in between;
   - electrical wiring **111** in the longitudinal body of the catheter **105** extending from the distal end to the proximal end, and adapted at the proximal end for connection to the electric power source **112**;
   - an emitter coil **110** at or near the distal end of the catheter **105**, connected at or near the distal end of the catheter **105** to the electrical wiring **111**, and adapted for energy emittance from a position in a patient's body near the implant device **101**, preferably surrounded by the implant device **101**;
   - an inflatable perfusion balloon **106** surrounding the emitter coil **110** comprising:
      - an evacuated balloon state adapted for insertion into the pulmonary vein **319**;
      - an expanded balloon state adapted for substantially preventing blood to reach the ablation region **302** of the implant device **301** and/or the treatment zone at the level of the antrum of the pulmonary vein **319** when the balloon **306** is in the expanded balloon state and positioned in the implant device **301** in the pulmonary vein **319**;
      - an exterior balloon surface;
      - an interior balloon chamber; and
      - a perfusion channel,
   - an inflation lumen **107** inside the longitudinal body, extending from the distal end to the proximal end of the catheter **105**, whereby the inflation lumen **107** is at the distal end of the catheter **105** in communication with the interior balloon chamber by means of an opening **108** in the catheter shaft, and whereby the inflation lumen **107** is adapted for connection with the pressurizing means **109** at the proximal end; and
   - a protector sheath **523** adapted for covering the implant device **501** in its compacted state upon insertion of the catheter **105** into a patient's body.

Pulmonary vein isolation comprises the steps of:
- providing abovementioned preferred embodiment of the system of the present invention;
- inserting the catheter in a patient's body, whereby the balloon is in the evacuated balloon state, and the implant device is in the compacted state, and whereby the implant device is covered with the protector sheath (**FIG. 5**);
- guiding the distal end of the catheter to the intended treatment zone in the pulmonary vein at the level of the antrum;
- retracting the protector sheath, which allows the self-expandable implant device to change from its compacted state to its expanded state, whereby the expanded implant device is deployed and attached onto the wall of the pulmonary vein;
- inflating the perfusion balloon with the pressurizing means, whereby the emitter coil inside the balloon is positioned inside the ablation region of the implant device, and whereby the axis of the emitter coil and the implant central axis are aligned substantially coaxial, and whereby the ablation region of the implant device and the treatment zone of the pulmonary vein are insulated from the blood flow; and whereby limited blood flow is sustained by means of the perfusion channel in the perfusion balloon (**FIG. 3**);
- providing an alternating current to the emitter coil by means of the electric power source, whereby the emitter coil emits a time-varying magnetic field, and whereby the pick-up coil of the implant device picks up the time-varying magnetic field, and whereby a current is induced in the pick-up coil, and whereby the current causes the ablation region to heat due to Joule heating, and whereby a substantially circumferential lesion is created at the treatment zone due to the heat of the ablation region for obtaining a signal-blocking path;
- deflating the balloon; and
- withdrawing the catheter from the patient's body.

Due to the shape of the implant device, a substantially circumferential lesion can be easily created, thereby obtaining a signal-blocking path. The implant device further remains fixed in the pulmonary vein after the treatment, preventing stenosis and allowing to ablate a new lesion at or near an older lesion at a later moment in time. Furthermore, the inflated perfusion balloon allows to insulate the ablation region and/or the treatment zone during ablation, requiring less power and/or current from the electric power source for the treatment. Furthermore, the positioning of the emitter coil in the implant device, substantially reduces the loss of time-varying magnetic fields in bodily tissue.

## Claims

1. System for ablating tissue to create a signal-blocking path on an inner wall of a vessel, comprising:
- an implant device provided with an ablation region which is adapted for surface contact with the inner wall of the vessel, the ablation region adapted for subtending at least a substantially complete circumferential band for ablating tissue to create a signal-blocking path at a treatment zone in the vessel upon heating of the ablation region;
- energy-providing means configured to provide the implant device with energy for heating up the ablation region,
wherein the system comprises:
- blood-flow interrupting means for temporarily interrupting blood from flowing along the ablation region of the implant device and/or along the treatment zone, thereby limiting heat being transferred away from the ablation region and/or treatment zone, **characterised in that** the blood-flow interrupting means comprises in combination a rapid pacing means and a balloon catheter.

2. System according to claim 1, in which the implant device is configured for implantation in a pulmonary vein for achieving pulmonary vein isolation.

3. System according to claim 1 or 2, in which the blood-flow interrupting means comprises a pressurizing means and a balloon catheter, the balloon catheter comprising:
- a distal end, a proximal end, and a longitudinal body in between;
- one or more inflatable balloons at or near the distal end of the balloon catheter, each of the balloons comprising:
- an evacuated balloon state adapted for insertion into the vessel;
- an expanded balloon state adapted for at least partially closing off the vessel;
- an exterior balloon surface; and
- an interior balloon chamber;
- one or more inflation lumens inside the longitudinal body, each of the inflation lumens extending from the distal end to the proximal end of the balloon catheter, whereby each of the inflation lumens is at the distal end of the balloon catheter in communication with the interior balloon chamber of at least one balloon, and whereby the inflation lumens are adapted for connection with the pressurizing means at the proximal end;
preferably wherein at least one of the balloons comprises an anticoagulant coating.

4. System according to claim 3, in which the exterior balloon surface of at least one balloon is adapted for fully closing off the vessel when said balloon is in the expanded balloon state.

5. System according to claim 3, in which at least one balloon is a perfusion balloon.

6. System according to any one of claims 3 to 5, in which the exterior balloon surface of at least one balloon is adapted for substantially preventing blood to reach the ablation region of the implant device and/or the treatment zone of the vessel when said balloon is in the expanded balloon state and positioned at or near the ablation region of the implant device, preferably said blood-flow interrupting means configured such that at least one balloon can be positioned more distally than the implant device for at least partially blocking the blood flow stream-upwards.

7. System according to any one of claims 3 to 6, in which the implant device comprises a compacted state and an expanded state, and in which the balloon catheter comprises at least one uninflated balloon in the evacuated balloon state, and in which the implant device is in the compacted state, and in which said uninflated balloon is positioned inside the implant device in the compacted state, and whereby said uninflated balloon is adapted for expansion of the implant device from the compacted state to the expanded state upon sufficiently pressurizing the interior balloon chamber of said uninflated balloon, and wherein preferably the catheter comprises a movable protector sheath suitable for protection of the implant device in its compacted state, whereby the movable protector sheath can fully or partially cover the implant device in its compacted state, and whereby the movable protector sheath can be retracted to fully uncover the implant device.

8. System according to any one of the preceding claims, in which the rapid pacing means comprises a pacemaker and one or more pacing catheters, each of the pacing catheters comprising:
- a distal end, a proximal end, and a longitudinal body in between; - electrical wiring in the longitudinal body of the pacing catheter extending from the distal end to the proximal end, and adapted at the proximal end for connection to the pacemaker; and
- one or more electrodes at or near the distal end of the pacing catheter, connected at or near the distal end of the pacing catheter to the electrical wiring,
and whereby the pacemaker comprises a pacing output circuit adapted to deliver pacing pulses through at least one of the electrodes of at least one of the pacing catheters, when the pacing output circuit is connected to the electrical wiring of at least one of the pacing catheters at its proximal end.

9. System according to claim 8, in which the rapid pacing means comprises an additional reference electrode, preferably a surface electrode, even more preferably a skin patch electrode, connectable by electrical wiring to the pacemaker.

10. System according to any one of claims 3 to 7 and according to any one of claims 8 or 9, in which one of the pacing catheters and the balloon catheter partially or fully coincide.

11. System according to any one of claims 3 to 7 and according to any one of claims 8 or 9, in which none of the pacing catheters coincide with the balloon catheter.

12. System according to any one of claims 1 to 11, in which the energy-providing means comprises an emitter coil, the implant device comprises a pick-up coil, and the system is adapted for wireless energy transfer from the emitter coil to the pick-up coil by means of time-varying magnetic fields.

13. System according to claim 12, in which the system is adapted for wireless energy transfer from the emitter coil to the pick-up coil when the energy- providing means is positioned outside a patient's body and the implant device is positioned near the treatment zone inside the vessel in the patient's body.

14. System according to claim 12, in which the energy-providing means comprises an electric power source and a coil catheter, the coil catheter comprising:
- a distal end, a proximal end, and a longitudinal body in between;
- electrical wiring in the longitudinal body of the coil catheter extending from the distal end to the proximal end, and adapted at the proximal end for connection to the electric power source; and
- the emitter coil at or near the distal end of the coil catheter, connected at or near the distal of the coil catheter to the electrical wiring of the coil catheter, and adapted for energy emittance from a position in a patient's body near the implant device, preferably surrounded by the implant device.

15. System according to any one of claims 3 to 7 and according to claim 14, in which the coil catheter and the balloon catheter partially or fully coincide, preferably the coil catheter and the balloon catheter fully coincide, more preferably the coil catheter and the balloon catheter fully coincide with the emitter coil positioned in the immediate vicinity of at least one of the balloons, and even more preferable the coil catheter and the balloon catheter fully coincide with one of the balloons surrounding the emitter coil.

## Patentansprüche

1. System zum Ablatieren von Gewebe, um einen signalsperrenden Weg an einer Innenwand eines Gefäßes zu erzeugen, Folgendes umfassend:
- ein Implantat, versehen mit einem Ablationsbereich, der für den Oberflächenkontakt mit der Innenwand des Gefäßes eingerichtet ist, wobei der Ablationsbereich dafür eingerichtet ist, mindestens ein im Wesentlichen vollständiges umlaufendes Band zum Ablatieren von Gewebe entgegenzusetzen, um auf das Erwärmen des Ablationsbereichs hin einen signalsperrenden Weg an einer Behandlungszone in dem Gefäß zu erzeugen,
- Energiebereitstellungsmittel, die dafür gestaltet sind, das Implantat für das Erwärmen des Ablationsbereichs mit Energie zu versorgen,
wobei das System Folgendes umfasst:
- Blutflussunterbrechungsmittel zum zeitweiligen Unterbrechen des Blutflusses entlang des Ablationsbereichs des Implantats und/oder entlang der Behandlungszone, wodurch das Abführen von Wärme von dem Ablationsbereich und/oder der Behandlungszone beschränkt wird, **dadurch gekennzeichnet, dass** die Blutflussunterbrechungsmittel in Kombination ein Rapid-Pacing-Mittel und einen Ballonkatheter umfassen.

2. System nach Anspruch 1, wobei das Implantat für die Implantation in eine Pulmonalvene gestaltet ist, um eine Isolation der Pulmonalvene zu erreichen.

3. System nach Anspruch 1 oder 2, wobei das Blutflussunterbrechungsmittel ein Druckbeaufschlagungsmittel und einen Ballonkatheter umfasst, wobei der Ballonkatheter Folgendes umfasst:
- ein distales Ende, ein proximales Ende und einen länglichen Körper dazwischen,
- einen oder mehrere aufblasbare Ballons am oder nahe dem distalen Ende des Ballonkatheters, wobei jeder der Ballons Folgendes umfasst:
- einen ausgeleerten Zustand des Ballons, der für das Einführen in das Gefäß eingerichtet ist,
- einen ausgeweiteten Zustand des Ballons, der für das zumindest teilweise Versperren des Gefäßes eingerichtet ist,
- eine Ballonaußenfläche und
- eine Balloninnenkammer,
- ein oder mehrere Aufpumplumen im Inneren des länglichen Körpers, wobei sich jedes der Aufpumplumen von dem distalen Ende zu dem proximalen Ende des Ballonkatheters erstreckt, wobei jedes der Aufpumplumen am distalen Ende des Ballonkatheters in Verbindung mit der Balloninnenkammer mindestens eines Ballons steht und wobei die Aufpumplumen für die Verbindung mit dem Druckbeaufschlagungsmittel am proximalen Ende stehen,
wobei mindestens einer der Ballons vorzugsweise eine Gerinnungshemmerbeschichtung umfasst.

4. System nach Anspruch 3, wobei die Ballonaußenfläche mindestens eines Ballons dafür eingerichtet ist, das Gefäß vollständig zu verschließen, wenn sich der Ballon in dem ausgeweiteten Zustand des Ballons befindet.

5. System nach Anspruch 3, wobei mindestens ein Ballon ein Perfusionsballon ist.

6. System nach einem der Ansprüche 3 bis 5, wobei die Ballonaußenfläche des mindestens einen Ballons dafür eingerichtet ist, im Wesentlichen zu verhindern, dass Blut den Ablationsbereich des Implantats und/oder der Behandlungszone des Gefäßes erreicht, wenn sich der Ballon in dem ausgeweiteten Zustand des Ballons befindet und am oder nahe dem Ablationsbereich des Implantats befindet, wobei das Blutflussunterbrechungsmittel vorzugsweise derart gestaltet ist, dass mindestens ein Ballon distaler als das Implantat positioniert werden kann, um den Blutfluss stromab zumindest teilweise zu sperren.

7. System nach einem der Ansprüche 3 bis 6, wobei das Implantat einen komprimierten Zustand und einen ausgeweiteten Zustand umfasst und wobei der Ballonkatheter in dem ausgeleerten Zustand des Ballons mindestens einen nicht aufgepumpten Ballon umfasst und wobei sich das Implantat in dem komprimierten Zustand befindet und wobei der nicht aufgepumpte Ballon in dem komprimierten Zustand im Inneren des Implantats positioniert ist und wobei der nicht aufgepumpte Ballon für die Ausweitung des Implantats von dem komprimierten Zustand in den ausgeweiteten Zustand auf ausreichende Druckbeaufschlagung der Balloninnenkammer des nicht aufgepumpten Ballons hin eingerichtet ist und wobei der Katheter vorzugsweise eine bewegliche Schutzhülle umfasst, die zum Schutz des Implantats in seinem komprimierten Zustand geeignet ist, wobei die bewegliche Schutzhülle das Implantat in seinem komprimierten Zustand vollständig oder teilweise bedecken kann und wobei die bewegliche Schutzhülle zurückgezogen werden kann, um das Implantat vollständig zu enthüllen.

8. System nach einem der vorhergehenden Ansprüche, wobei das Rapid-Pacing-Mittel einen Schrittmacher und einen oder mehrere Schrittmacher-Katheter umfasst, wobei jeder der Schrittmacher-Katheter Folgendes umfasst:
- ein distales Ende, ein proximales Ende und einen länglichen Körper dazwischen,
- Elektroverdrahtung in dem länglichen Körper des Schrittmacher-Katheters, die sich vom distalen Ende zum proximalen Ende erstreckt und am proximalen Ende für eine Verbindung mit dem Schrittmacher eingerichtet ist, und
- eine oder mehrere Elektroden am oder nahe dem distalen Ende des Schrittmacher-Katheters, die am oder nahe dem distalen Ende des Schrittmacher-Katheters mit der Elektroverdrahtung verbunden sind,
und wobei der Schrittmacher eine Schrittmacher-Ausgabeschaltung umfasst, die dafür eingerichtet ist, Schrittmacher-Impulse durch mindestens eine der Elektroden mindestens eines der Schrittmacher-Katheter abzugeben, wenn die Schrittmacher-Ausgabeschaltung mit der Elektroverdrahtung mindestens eines der Schrittmacher-Katheter an dessen proximalem Ende verbunden ist.

9. System nach Anspruch 8, wobei das Rapid-Pacing-Mittel eine zusätzliche Referenzelektrode umfasst, vorzugsweise eine Oberflächenelektrode, noch bevorzugter eine Hautelektrode, die durch Elektroverdrahtung mit dem Schrittmacher verbunden werden kann.

10. System nach einem der Ansprüche 3 bis 7 und nach einem der Ansprüche 8 oder 9, wobei sich einer der Schrittmacher-Katheter und der Ballonkatheter teilweise oder vollständig decken.

11. System nach einem der Ansprüche 3 bis 7 und nach einem der Ansprüche 8 oder 9, wobei sich keiner der Schrittmacher-Katheter mit dem Ballonkatheter deckt.

12. System nach einem der Ansprüche 1 bis 11, wobei das Energiebereitstellungsmittel eine Emitterspule umfasst, das Implantat eine Antennenspule umfasst und das System für die drahtlose Energieübertragung von der Emitterspule zur Antennenspule mittels zeitlich variierender Magnetfelder eingerichtet ist.

13. System nach Anspruch 12, wobei das System zur drahtlosen Energieübertragung von der Emitterspule zur Antennenspule eingerichtet ist, wenn das Energiebereitstellungsmittel außerhalb eines Patientenkörpers positioniert ist und das Implantat nahe der Behandlungszone in dem Gefäß des Patientenkörpers positioniert ist.

14. System nach Anspruch 12, wobei das Energiebereitstellungsmittel eine Elektroenergiequelle und einen Spulenkatheter umfasst, wobei der Spulenkatheter Folgendes umfasst:
- ein distales Ende, ein proximales Ende und einen länglichen Körper dazwischen,
- Elektroverdrahtung in dem länglichen Körper des Spulenkatheters, die sich vom distalen Ende zum proximalen Ende erstreckt und am proximalen Ende für eine Verbindung mit der Elektroenergiequelle eingerichtet ist, und
- die Emitterspule am oder nahe dem distalen Ende des Spulenkatheters, die am oder nahe dem distalen Ende des Spulenkatheters mit der Elektroverdrahtung des Spulenkatheters verbunden und für eine Energieemittanz von einer Position in einem Patientenkörper nahe dem Implantat eingerichtet ist, vorzugsweise von dem Implantat umgeben.

15. System nach einem der Ansprüche 3 bis 7 und nach Anspruch 14, wobei sich der Spulenkatheter und der Ballonkatheter teilweise oder vollständig decken, sich der Spulenkatheter und der Ballonkatheter vorzugsweise vollständig decken, bevorzugter sich der Spulenkatheter und der Ballonkatheter vollständig mit der Emitterspule decken, die in der unmittelbaren Nähe mindestens eines der Ballons positioniert ist, und sich der Spulenkatheter und der Ballonkatheter noch bevorzugter vollständig mit einem der Ballons decken, der die Emitterspule umgibt.

## Revendications

1. Système d'ablation de tissu pour créer un chemin de blocage de signaux sur une paroi interne d'un vaisseau, comprenant :
- un dispositif d'implant pourvu d'une région d'ablation qui est conçue pour un contact de surface avec la paroi interne du vaisseau, la région d'ablation étant conçue pour sous-tendre au moins une bande circonférentielle sensiblement complète pour ablater un tissu pour créer un chemin de blocage de signaux au niveau d'une zone de traitement dans le vaisseau lors du chauffage de la zone d'ablation ;
- un moyen de fourniture d'énergie configuré pour fournir de l'énergie au dispositif d'implant pour le chauffage de la région d'ablation,
dans lequel le système comprend :
- un moyen d'interruption de flux sanguin pour empêcher temporairement le sang de circuler le long de la région d'ablation du dispositif d'implant et/ou le long de la zone de traitement, limitant ainsi le transfert de chaleur à partir de la région d'ablation et/ou de la zone de traitement, **caractérisé en ce que** le moyen d'interruption de flux sanguin comprend en combinaison un moyen de stimulation rapide et un cathéter à ballonnet.

2. Système selon la revendication 1, dans lequel le dispositif d'implant est configuré pour une implantation dans une veine pulmonaire pour réaliser l'isolation de la veine pulmonaire.

3. Système selon la revendication 1 ou 2, dans lequel le moyen d'interruption de flux sanguin comprend un moyen de mise sous pression et d'un cathéter à ballonnet, le cathéter à ballonnet comprenant :
- une extrémité distale, une extrémité proximale, et un corps longitudinal entre les deux ;
- un ou plusieurs ballonnets gonflables au niveau de ou à proximité de l'extrémité distale du cathéter à ballonnet, chacun des ballonnets comprenant :
- un état de ballonnet dégonflé adapté pour l'insertion dans le vaisseau ;
- un état de ballonnet gonflé adapté pour fermer au moins partiellement le vaisseau ;
- une surface extérieure de ballonnet ; et
- une chambre intérieure de ballonnet ;
- une ou plusieurs lumières de gonflage à l'intérieur du corps longitudinal, chacune des lumières de gonflage s'étendant à partir de l'extrémité distale vers l'extrémité proximale du cathéter à ballonnet, moyennant quoi chacune des lumières de gonflage est, au niveau de l'extrémité distale du cathéter à ballonnet, en communication avec la chambre intérieure de ballonnet d'au moins un ballonnet, et moyennant quoi les lumières de gonflage sont conçues pour une connexion au moyen de mise sous pression au niveau de l'extrémité proximale ;
de préférence dans lequel au moins l'un des ballonnets comprend un revêtement anticoagulant.

4. Système selon la revendication 3, dans lequel la surface extérieure de ballonnet d'au moins un ballonnet est conçue pour fermer complètement le vaisseau lorsque ledit ballonnet est dans l'état de ballonnet gonflé.

5. Système selon la revendication 3, dans lequel au moins un ballonnet est un ballonnet de perfusion.

6. Système selon l'une quelconque des revendications 3 à 5, dans lequel la surface extérieure de ballonnet d'au moins un ballonnet est conçue pour empêcher substantiellement le sang d'atteindre la région d'ablation du dispositif d'implant et/ou de la zone de traitement du vaisseau lorsque ledit ballonnet est dans l'état de ballonnet gonflé et positionné au niveau de ou à proximité de la région d'ablation du dispositif d'implant, de préférence, ledit moyen d'interruption de flux sanguin est configuré de telle sorte qu'au moins un ballonnet peut être positionné de manière plus distale que le dispositif d'implant pour bloquer au moins partiellement la circulation sanguine vers le haut.

7. Système selon l'une quelconque des revendications 3 à 6, dans lequel le dispositif implant comprend un état comprimé et un état expansé, et dans lequel le cathéter à ballonnet comprend au moins un ballonnet non gonflé dans l'état de ballonnet dégonflé, et dans lequel le dispositif d'implant est dans l'état comprimé, et dans lequel ledit ballonnet non gonflé est placé à l'intérieur du dispositif d'implant dans l'état comprimé, et dans lequel ledit ballonnet non gonflé est conçu pour l'expansion du dispositif d'implant de l'état comprimé à l'état expansé lorsque la chambre intérieure de ballonnet dudit ballonnet non gonflé reçoit une pression suffisante, et dans lequel de préférence, le cathéter comprend une gaine de protection mobile appropriée pour la protection du dispositif d'implant dans son état comprimé, moyennant quoi la gaine de protection mobile peut recouvrir entièrement ou partiellement le dispositif d'implant dans son état comprimé, et dans lequel la gaine de protection mobile peut être rétractée pour découvrir entièrement le dispositif d'implant.

8. Système selon l'une quelconque des revendications précédentes, dans lequel le moyen de stimulation rapide comprend un stimulateur cardiaque et un ou plusieurs cathéters de stimulation, chacun des cathéters de stimulation comprenant :
- une extrémité distale, une extrémité proximale, et un corps longitudinal entre les deux ;
- un câblage électrique dans le corps longitudinal du cathéter de stimulation allant de l'extrémité distale à l'extrémité proximale, et étant conçu au niveau de l'extrémité proximale pour la connexion au stimulateur cardiaque ; et
- une ou plusieurs électrodes au niveau ou à proximité de l'extrémité distale du cathéter de stimulation, connectées au niveau de ou à proximité de l'extrémité distale du cathéter de stimulation au câblage électrique,
et dans lequel le stimulateur cardiaque comprend un circuit de sortie de stimulation conçu pour délivrer des impulsions de stimulation à travers au moins une des électrodes d'au moins l'un des cathéters de stimulation, lorsque le circuit de sortie de stimulation est connecté au câblage électrique d'au moins l'un des cathéters de stimulation au niveau de son extrémité proximale.

9. Système selon la revendication 8, dans lequel le moyen de stimulation rapide comprend une électrode de référence supplémentaire, de préférence une électrode de surface, encore plus préférablement une électrode de timbre transdermique, pouvant être connectée par un câblage électrique au stimulateur cardiaque.

10. Système selon l'une quelconque des revendications 3 à 7 et selon l'une quelconque des revendications 8 ou 9, dans lequel l'un des cathéters de stimulation et le cathéter à ballonnet coïncident partiellement ou totalement.

11. Système selon l'une quelconque des revendications 3 à 7 et selon l'une quelconque des revendications 8 ou 9, dans lequel aucun des cathéters de stimulation ne coïncide avec le cathéter à ballonnet.

12. Système selon l'une quelconque des revendications 1 à 11, dans lequel le moyen de fourniture d'énergie comprend une bobine émettrice, le dispositif d'implant comprend une bobine détectrice, et le système est conçu pour le transfert d'énergie sans fil à partir de la bobine émettrice vers la bobine détectrice au moyen de champs magnétiques variant dans le temps.

13. Système selon la revendication 12, dans lequel le système est conçu pour le transfert d'énergie sans fil à partir de la bobine émettrice vers la bobine détectrice lorsque le moyen de fourniture d'énergie est positionné à l'extérieur du corps d'un patient et le dispositif d'implant est positionné à proximité de la zone de traitement à l'intérieur du vaisseau dans le corps du patient.

14. Système selon la revendication 12, dans lequel le moyen de fourniture d'énergie comprend une source d'énergie électrique et un cathéter « queue de cochon », le cathéter « queue de cochon » comprenant :
- une extrémité distale, une extrémité proximale, et un corps longitudinal entre les deux ;
- un câblage électrique dans le corps longitudinal du cathéter « queue de cochon » allant de l'extrémité distale à l'extrémité proximale, et conçu au niveau de l'extrémité proximale pour la connexion à la source d'alimentation électrique; et
- la bobine émettrice au niveau de ou à proximité de l'extrémité distale du cathéter « queue de cochon », connectée au niveau de ou à proximité de l'extrémité distale du cathéter «queue de cochon» au câblage électrique du cathéter « queue de cochon », et conçue pour l'émittance d'énergie à partir d'une position dans le corps d'un patient à proximité du dispositif d'implant, de préférence entourée par le dispositif de l'implant.

15. Système selon l'une quelconque des revendications 3 à 7 et selon la revendication 14, dans lequel le cathéter « queue de cochon » et le cathéter à ballonnet coïncident partiellement ou entièrement, de préférence, le cathéter « queue de cochon » et le cathéter à ballonnet coïncident entièrement, plus préférablement le cathéter « queue de cochon » et le cathéter à ballonnet coïncident entièrement avec la bobine émettrice positionnée au voisinage immédiat d'au moins l'un des ballonnets, et encore plus préférablement le cathéter « queue de cochon » et le cathéter à ballonnet coïncident entièrement avec l'un des ballonnets entourant la bobine émettrice.
